# EUROPEAN PATENT APPLICATION

(11) **EP 4 775 224 A1**
(43) Date of publication of application: **15.07.2026**
(21) Application number: 23951601.6
(22) Date of filing: 08.09.2023
(51) Int. Cl.: A61K 47/65, A61K 47/54, A61K 31/704, C07B 63/04

(54) **METHOD FOR RECOVERING AND REMOVING PALLADIUM IN PREPARATION OF ANTICANCER AGENT PRODRUG CONJUGATE**

(71) Applicant: Pharosgen Co., Ltd., Seoul 05852 (KR)
(72) Inventor: BYUN, Young Ro, Seoul 01318 (KR); KIM, Sang Yoon, Seoul 06362 (KR); KIM, Sang Hwan, Gyeonggi-do 13523 (KR); PARK, Geon Tae, Seoul 04186 (KR); KO, Yoon Gun, Seoul 04375 (KR); LEE, Hye Min, Seoul 08599 (KR); HWAG, Jae Taeg, Suwon-si Gyeonggi-do 08599 (KR); KIM, Byung Ok, Asan-si Chungcheongnam-do 31579 (KR); LEE, Yong Ki, Wonju-si Gangwon-do 26425 (KR); KWON, Young Ho, Asan-si Chungcheongnam-do 31528 (KR); KIM, Sung Bin, Cheonan-si Chungcheongnam-do 31170 (KR); YUN, Yeo Jeong, Asan-si Chungcheongnam-do 31515 (KR)
(74) Representative: Valenza, Silvia
(86) International application number: PCT/KR2023/013542
(87) International publication number: WO 2025/053315

(57) **Abstract**

The present invention provides a method for effectively removing and recovering residual palladium in the drug through a simple process improvement in the preparation of maleimide-KGDEVD-PABC-doxorubicin, a caspase-activated anticancer prodrug conjugate.

## Description

### TECHNICAL FIELD

The present invention relates to a method for the recovery and removal of palladium from a preparation process of anticancer prodrug conjugates, and more particularly to a method for the recovery and removal of palladium from the preparation of a caspase-activated anticancer drug prodrug conjugate.

### BACKGROUND ART

Anti-cancer chemotherapeutic agents or anticancer drugs are the most commonly used in cancer treatment due to their potent anti-cancer effects, but their administration is often limited by severe side effects and toxicity. Therefore, there is a need to make the anti-cancer chemotherapeutic agents selectively act on cancerous tissues without acting on normal tissues. To this end, antibodies or peptides that recognize and bind to biomarkers that are not or rarely expressed in normal cells but are specifically expressed in tumor cells have been commonly applied to anticancer chemotherapeutic agents for their selective delivery to cancer tissues. However, as the genetic signature of each patient differs even within the same cancer type, these drugs are limited to patients with the cancers expressing the biomarkers that can be targeted by the drugs, making them inherently inaccessible to a wide range of patients. In addition, recent studies have revealed intratumor heterogeneity, meaning that different tumor cells within the same cancer tissue can have varying genotypes. Therefore, biopsies that are routinely performed to characterize a cancer may not accurately represent the genetic profile of the entire tumor. This means that even if the biomarker targeted by the drug is detected through a biopsy, the effectiveness of the drug remains uncertain. Conventional drugs have fundamental limitations in delivering chemotherapeutic agents to all tumor cells, making them unsuitable as an ideal drug delivery system for chemotherapy and ineffective for the application of effective cancer treatment. To address these issues, prodrugs that can be specifically activated within tumor cells and cancer tissues have been developed. In this regard, Korean Reference Patent No. 1759261 discloses a multifunctional anticancer prodrug activated by inducible genes, its method of preparation, and its use. The method of preparing the prodrug described in the above prior art involves removing the allyl group of maleimide-KGDEVD-PABC-doxorubicin and adding tetrakis(triphenylphosphine)palladium (Pd(PPh₃)₄) and acetic acid to induce deprotection.

### DETAILED DESCRIPTION OF THE INVENTION

### TECHNICAL PROBLEM

However, in the above prior art, a large amount of palladium remains due to co-precipitation during the dissolution and crystallization process. Various methods such as washing-separation, resin treatment, and filtration are used to remove the palladium, but they are inadequate to remove the palladium to the desired level.

The present invention aims to solve the above problems and others, and provide a method for effectively removing and recovering residual palladium in the drug through a simple process improvement in the preparation method of maleimide-KGDEVD-PABC-doxorubicin, which is a caspase-activated anticancer prodrug conjugate.

### TECHNICAL SOLUTION

In one aspect of the present invention, there is provided a method of removing and recovering high purity palladium compounds from the preparation process of prodrug conjugates, comprising; preparing an allyl group-protected precursor of a prodrug conjugate in which an anticancer compound linked to a peptide linker that is to be cleaved by a proteolytic enzyme;
preparing a deprotected reaction solution in which the allyl groups of the precursor have been removed by dissolving the precursor and tetrakis triphenylphosphine palladium (Pd(PPh₃)₄) in an anhydrous organic solvent;
removing bis(triphenylphosphine)palladium dichloride (PdCl₂₍(PPh₃)₂) prepared by a reaction between Pd(PHh₃)₄ and hydrochloric acid by adding hydrochloric acid and a catalyst to the deprotected reaction solution, stirring at room temperature, and filtering a reactant; and
precipitating an amorphous solid by adding a mixture including an organic solvent and a precipitating solvent to the deprotected reaction solution in which PdCl₂₍(PPh₃)₂ is removed.

### EFFECT OF THE INVENTION

The method for the recovery and removal of palladium from the preparation of the anticancer prodrug (drug precursor) conjugates of the present invention as described above employs a simple process improvement to realize the fast recovery and removal of residual palladium in the drug used for the deprotection reaction removing allyl groups. Compared to the prior art methods, the method of the present invention is economical due to the simplified separation and purification process and the high removal efficiency the increases the yield to 35%, therefore, it can be utilized in a mass production process to produce low-cost and high-quality prodrugs. However, these effects do not limit the scope of the present invention.

### BRIEF DESCRIPTION OF THE DRAWINGS

FIG. 1 is a graph showing the results of IR analysis of Pd(PPh₃)₄ STD as a by-product filtrate from the production process of the present invention.
FIG. 2 is a graph showing the results of IR analysis of PdCl₂(PPh₃)₂ STD as a by-product filtrate from the production process of the present invention.
FIG. 3 is a graph showing the results of IR analysis of the Pd filtrate as a by-product of the production process of the present invention.
FIG. 4 is a graph showing the results of IR analysis of Pd(PPh₃)₄ STD as a by-product filtrate from the production process of the present invention.
FIG. 5 is a graph showing the results of IR analysis of PdCl₂(PPh₃)₂ STD as a by-product filtrate from the production process of the present invention.
FIG. 6 is a graph showing the results of IR analysis of the Pd filtrate as a by-product of the production process of the present invention.
FIG. 7 is a graph showing the results of P-NMR analysis of Pd(PPh₃)₄ STD as a by-product filtrate from the production process of the present invention.
FIG. 8 is a graph showing the results of P-NMR analysis of PdCl₂(PPh₃)₂ STD as a by-product filtrate from the production process of the present invention.
FIG. 9 is a graph showing the results of P-NMR analysis of a Pd filtrate as a by-product filtrate of the production process of the present invention.
FIG. 10 is an illustration of the chemical structure of maleimide-KGDEVD-PABC-doxorubicin of the present invention.
FIG. 11 is a schematic representation showing the reaction scheme of the preparation of a prodrug by conventional methods, wherein the prodrug comprises a peptide spacer (KGDEVD, sequence number 2) with one maleimide functional group and Asp-Glu-Val-Asp sequence (DEVD, sequence number 1), and doxorubicin as the active ingredient. Acetic acid (AcOH) is used between the reaction of doxorubicin and the deprotection of the allylic group of the peptide in the peptide spacer. Doxorubicin and the peptide spacer are conjugated with maleimide-KGDEVD-PABC-doxorubicin via p-aminobenzyl carbamate (PABC).

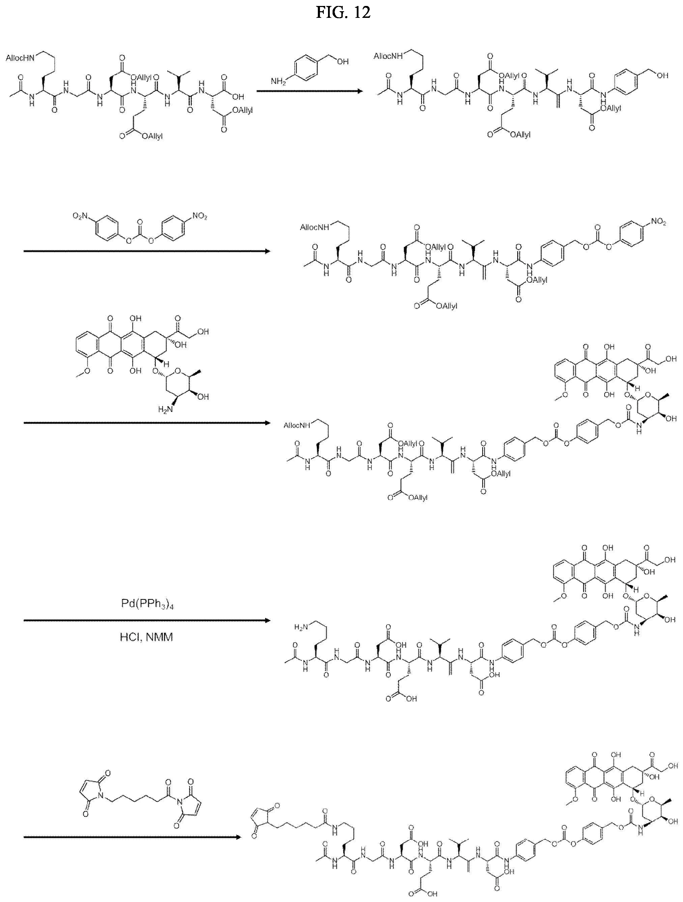

FIG. 12 is a schematic diagram illustrating the production process of the prodrugs of the present invention. It shows the reaction scheme for the preparation of a prodrug comprising one maleimide functional group, a peptide spacer having the sequence Asp-Glu-Val-Asp (DEVD, sequence no. 1), and doxorubicin as the active ingredient. Hydrochloric acid (HCl) is used between the reaction of doxorubicin and the deprotection of the allylic group of the peptide in the peptide spacer. The doxorubicin and peptide spacer are conjugated with maleimide-KGDEVD-PABC-doxorubicin via p-aminobenzylcarbamate (PABC).
FIG. 13 is a schematic representation showing the reaction of tetrakis triphenylphosphine palladium to become bis triphenylphosphine palladium dichloride in a dimethylformamide solvent containing hydrochloric acid.

### BEST MODE FOR THE INVENTION

### Definitions of terms:

As used herein, the term "palladium" (symbol Pd) refers to a white metal used in catalysts and specialty alloys that is a good adsorber of hydrogen. In 1802, palladium was isolated along with rhodium from platinum-bearing ores in South America by the Englishman Wollaston. Palladium, atomic number 46, is a white metal like ruthenium (Ru) and rhodium (Rh), usually found in platinum ores as a form of natural alloys. Although rare, they are highly useful for various applications, such as catalysts in catalytic converters for automobile emissions and in the chemical industry, or precious metals used in jewelry either on their own (including alloys) or as plating. Palladium, along with Pt, Rh, and Ru, is listed as a route-dependent human toxicant and must be controlled at 10 ppm or less in pharmaceuticals.

As used herein, the term "prodrug" refers to a pre-metabolized drug that is inactive *in vitro,* but is metabolized by metabolic enzymes to act as a drug when it enters the body. This involves a change of the chemical structure of the drug so that it can have an effect on the desired target.

### A detailed description of the invention:

In one aspect of the present invention, there is provided a method of removing and recovering high purity palladium compounds from the preparation process of prodrug conjugates, comprising; preparing an allyl group-protected precursor of a prodrug conjugate in which an anticancer compound linked to a peptide linker that is to be cleaved by a proteolytic enzyme;
preparing a deprotected reaction solution in which the allyl groups of the precursor have been removed by dissolving the precursor and tetrakis triphenylphosphine palladium (Pd(PPh₃)₄) in an anhydrous organic solvent;
removing bis(triphenylphosphine)palladium dichloride (PdCl₂₍(PPh₃)₂) prepared by a reaction between Pd(PHh₃)₄ and hydrochloric acid by adding hydrochloric acid and a catalyst to the deprotected reaction solution, stirring at room temperature, and filtering a reactant; and
precipitating an amorphous solid by adding a mixture including an organic solvent and a precipitating solvent to the deprotected reaction solution in which PdCl₂₍(PPh₃)₂ is removed.

The above method may further comprise purifying the prodrug conjugate by adding the obtained prodrug conjugate to a reaction solution comprising triphenylphosphine, hydrochloric acid and a precipitating solvent, wherein the prodrug conjugate may be selected from the group comprising maleimide-KGDEVD-PABC-doxorubicin and the catalyst may be 4-methylmorpholine, morpholine, 4-ethylmorpholine, pyrrolidine, properidine, N-methylpiperidine, N-methylpyrrolidine, piperazine, and imidazolidine.

In the above method, the precipitating solvent may be dichloromethane or chloroform, and the anhydrous organic solvent may be dimethylformamide, dichloromethane, or chloroform, and the organic solvent may be ethanol, methanol, or acetone.

The present invention relates to a method of preparing a caspase-activated anticancer prodrug conjugate, maleimide-KGDEVD-PABC-doxorubicin. As mentioned above, the methods of preparing prodrugs described in the prior art are based on removing the allyl group of Ac-Lys(OAloc)-Gly-Asp(OAll)-Glu(OAll)-Val-Asp(OAll)-PABC-DOX and inducing a deprotection reaction with the addition of tetrakis(triphenylphosphine)palladium (Pd(PPh₃)₄) and acetic acid, which makes Pd(PPh₃)₄ dissolve and a large amount co-precipitate during crystallization, leading to a residual palladium content in the final product of 4,000-5,000 ppm beyond the standard. Subsequently, the N-(ε-maleimidocaproxyloxy)succinimide ester (EMCS) coupling reaction occurs to prepare EMC-AcKGDEVD-PABC-DOX, but the palladium is not removed at all from the mother liquor. Even after separation and purification using Prep HPLC to obtain high purity Ac-Lys(EMC)-Gly-Asp-Glu-Val-Asp-PABC-doxorubicin (red amorphous solid), 100-300 ppm of palladium remains. Although the palladium can be reduced to below the standard of 6.6 ppm through repeating the above separation and purification process at least three times, the recovery yield for a single purification process is 35%, so the overall yield after three repetitions drops below 5%, making it unsuitable as a method for removing palladium. Existing methods for removing palladium (Pd) compounds include distillation, recrystallization, adsorption using carbon black, and silica gel column chromatography, but since they cannot remove palladium to a low level (10 ppm or less), they have low removal efficiency and high cost, which makes them inefficient for mass production (see Tables 1 to 4).

The present inventors found that the reason for the residual palladium in maleimide-KGDEVD-PABC-doxorubicin is the dissolution of Pd(PPh₃)₄ during the deprotection reaction by removing the allyl group and its co-precipitation during crystallization. Therefore, the present inventors introduced a simple process improvement to prevent palladium from remaining in the drug, and developed a method to effectively remove and recover palladium through a simple process. It was realized by replacing the reaction reagent from acetic acid to hydrochloric acid (HCl), so that Pd(PPh₃)₄ and hydrochloric acid react to produce PdCl₂(PPh₃)₂ which has totally different properties. The problem of residual palladium was solved by selectively crystallizing and filtering the by-products.

Various studies have been conducted to make chemotherapeutic agents selectively act on tumor tissue but not on normal tissue, including the studies on recognition of biomarkers specifically expressed by tumor cells. However, even if tumor cells expressing the target biomarker account for a majority of the total tumor cells in the cancer tissue, the remaining tumor cells are not affected by the drug and therefore cause cancer growth even after the treatment. Thus, conventional drugs have fundamental limitations in delivering drugs to all developed tumor cells, making them inadequate delivery systems for ideal anticancer chemotherapeutics and inappropriate for effective cancer treatment. Furthermore, when most cancer tissues express target molecules, anticancer chemotherapeutic agents affect other cells in close proximity to or in contact with the cancer tissue, causing side effects or toxicity. To address these problems, the present inventors developed a prodrug that can be specifically activated within tumor cells and cancer tissue. For example, U.S. Patent No. 7,445,764 discloses a conjugate of a peptide and doxorubicin anticancer agent, cleavable matrix metalloproteinase (MMP). In addition, U.S. Patent Publication No. 2010/0111866 discloses a prodrug conjugate in the form of maleimide-hydrazone-doxorubicin, and U.S. Patent Publication No. 2013/0338422 discloses a prodrug conjugate in the form of peptide sequence-DEVD-doxorubicin. However, these conjugates still suffer from one or more of the following issues: low potency, low selectivity, and/or frequent dosing.

Recognizing these problems, the present inventors strived to develop a prodrug-like anticancer chemotherapeutic agent that selectively acts on tumor cells but doesn't affect normal tissues, with minimal side effects and excellent efficacy. Accordingly, the present inventors developed a caspase-activated anticancer prodrug conjugate, maleimide-KGDEVD-PABC-doxorubicin. It is a prodrug in which a thiol group and a bindable maleimide group are derived at the N-terminus of an Asp-Glu-Val-Asp peptide (sequence number 1) and an anticancer chemotherapeutic agent, especially doxorubicin as an active ingredient for an anticancer drug, is chemically bound at the C-terminus. After intravenous administration, the anticancer chemotherapeutic agent doxorubicin binds to a linker, which has a maleimide group that binds to the thiol group of endogenous albumin and an Asp-Glu-Val-Asp peptide sequence (sequence number 1) that is enzymatically hydrolyzed by caspase 3 which is activated in irradiated tumors. After that, the substance is delivered to the tumor tissue by endogenous albumin, and then doxorubicin is released by caspase 3 to exert pharmacological effects.

In the preparation of the above drug, the first step involves a reaction of Ac-Lys(OAlloc)-Gly-Asp(OAll)-Glu(OAll)-Val-Asp(OAll)-OH, with 4-aminobenzyl alcohol, and EEDQ under DMF solvent to obtain Ac-Lys(OAloc)-Gly-Asp(OAll)-Glu(OAll)-Val-Asp(OAll)-PABOH. Then it reacts with 4-nitrophenyl chloroformate, 2,6-rutidine in anhydrous dichloromethane solvent to obtain Ac-Lys(OAloc)-Gly-Asp(OAll)-Glu(OAll)-Val-Asp(OAll)-PABC. After that, it reacts with doxorubicin hydrochloride and DIEA in DMF solvent to obtain Ac-Lys(OAloc)-Gly-Asp(OAll)-Glu(OAll)-Val-Asp(OAll)-PABC-DOX. Subsequently, it reacts with Pd(PPh₃)₄ and acetic acid under DMF solvent to induce deprotection, and the resulting Ac-Lys-Gly-Asp-Glu-Val-Asp-PABC-DOX (red amorphous solid) is isolated. Then it reacts with N-(ε-Ramidocaproxyloxy)succinimide ester and triethylamine under DMF solvent and separated/purified with ODS-A 5 µm Resin Column to obtain Ac-Lys(EMC)-Gly-Asp-Glu-Val-Asp-PABC-doxorubicin. The separation/purification process is repeated at least three times to remove palladium.

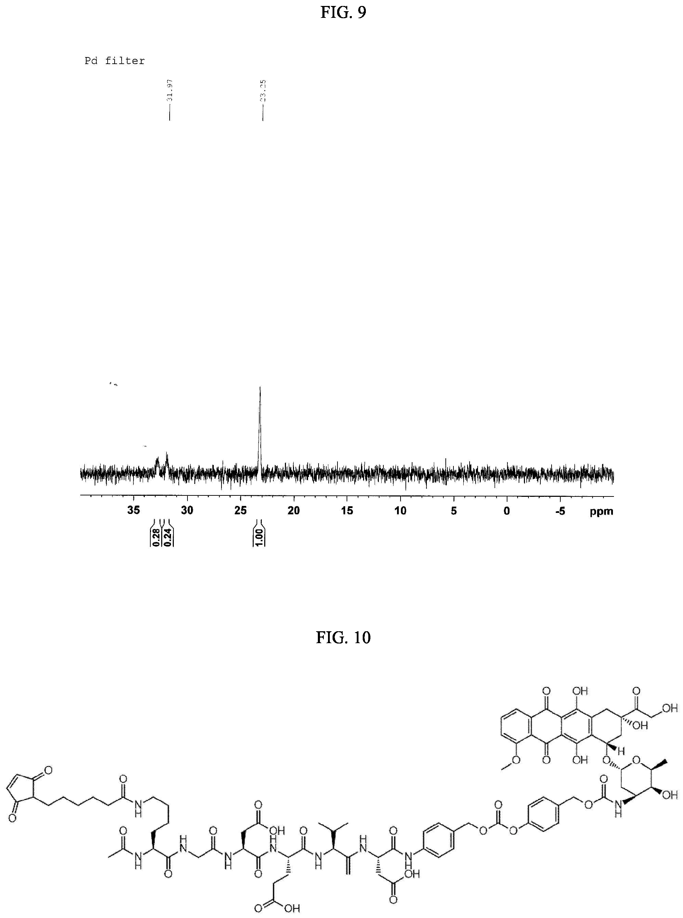

In conclusion, the present invention relates to a method of preparing maleimide-KGDEVD-PABC-doxorubicin, wherein the deprotection reaction of Ac-Lys(OAloc)-Gly-Asp(OAll)-Glu(OAll)-Val-Asp(OAll)-PABC-DOX is used to obtain Ac-Lys(EMC)-Gly-Asp-Glu-Val-Asp-PABC-doxorubicin (FIG. 10). During the reaction, Pd(PPh₃)₄ and hydrochloric acid react to give PdCl₂(PPh₃)₂, and the resulting side product selectively crystallizes under dimethylformamide solvent and can be easily removed by filtration. The recovered side product is a light yellow, high purity product that can be reused in other reactions. The PdCl₂(PPh₃)₂ is then crystallized by injection of methane dichloride and filtered to obtain the desired compound. The palladium content of the as-obtained product is 60 ppm. Subsequently, trace amounts of triphenylphosphine and hydrochloric acid are added, followed by slurrying without dissolution and filtering, and the residual palladium is removed to a level of 11 ppm in the mother liquor. As a result, the present inventors found that the residual palladium is significantly reduced from 4,000 to 5,000 ppm to 20 ppm or less.

Another method of palladium removal is to dissolve Ac-Lys(OAloc)-Gly-Asp(OAll)-Glu(OAll)-Val-Asp(OAll)-PABC-DOX in dimethylformamide, then add Pd(PPh₃)₄ and hydrochloric acid for deprotection reaction, followed by filtration to remove PdCl₂(PPh₃)₂. Then it is crystallized using chloroform and filtered to obtain the desired compound. The palladium that still has not been removed by filtration is removed from the mother liquor, and the as-obtained palladium residue in the final desired compound is less than 13 ppm. If acetic acid is used as a reagent, Ac-Lys(OAloc)-Gly-Asp(OAll)-Glu(OAll)-Val-Asp(OAll)-PABC-DOX is dissolved in dimethylformamide, Pd(PPh₃)₄ and acetic acid are added for deprotection reaction, and then crystallized using chloroform and filtered to obtain the desired compound. The residual palladium is removed with the mother liquor, and the as-obtained palladium residue in the final desired compound is less than 100 ppm. The prior art has the disadvantage that it requires multiple separation and purification processes to remove Pd(PPh₃)₄, resulting in a very demanding process and a low recovery rate of 5%, raising the production unit cost. However, as described above, the recovery and removal method of palladium of the present invention simplifies the separation process and purification process and increases the yield to 35%, thus enabling low-cost and high-quality prodrug production.

The present invention will now be described in more detail with reference to the following examples. However, the invention is not limited to the embodiments disclosed herein, but may be embodied in many different forms, and the following embodiments are provided to make the disclosure of the invention complete and to give those of ordinary skill in the art a complete idea of the scope of the invention.

### Reference Example 1: Preparation of Maleimide-Lys-Asp-Glu-Val-Asp-PABC-Doxorubicin Prodrug

For the preparation of the above prodrug, Ac-Lys(OAloc)-Gly-Asp(OAll)-Glu(OAll)-Val-Asp(OAll)-OH (344 mg, 0.38 mmol), 4-aminobenzyl alcohol (2 eq), and EEDQ (2 eq) were dissolved in anhydrous DMF (11 mL) and stirred at room temperature under argon gas for 24 hours. The solution was then concentrated in vacuum and crystallized from ether to give Ac-Lys(OAloc)-Gly-Asp(OAll)-Glu(OAll)-Val-Asp(OAll)-PABOH. Then, the above Ac-Lys(OAloc)-Gly-Asp(OAll)- Glu(OAll)-Val-Asp(OAll)-PABOH (322 mg, 0.318 mmol) and 4-nitrophenyl chloroformate (1.2 eq) were dissolved in anhydrous dichloromethane (10 mL), followed by the addition of 2,6-rutidine (3 eq) and stirred at room temperature. After 2 h, anhydrous DMF (2 mL) and 2,6-rutidine (2 eq) was added. After 24, 27 and 46 h, 2,6-rutidine (4.75 eq), 4-nitrophenyl chloroformate (1 eq) were added respectively and the reaction was stirred at room temperature. After 84 h, an aqueous solution of sodium bicarbonate was added to quench the reaction. The reaction was then extracted three times with ethyl acetate and the organic layer was successively washed with 0.5 M citric acid, aqueous NaHCO₃, and brine. The obtained organic layer was dried over anhydrous NaSO₄, filtered, and concentrated under reduced pressure. The concentrate was then purified by semi-preparative HPLC (water/CH₃CN, containing 0.1% TFA as an additive, CH₃CN 20-100% over 50 min, 10 ml/min) using a C18 reverse phase column (250 mm X 22 mm) to afford Ac-Lys(OAloc)-Gly-Asp(OAll)- Glu(OAll)-Val-Asp(OAll)-PABC (77 mg, 20.5%). ESI-MS: m/z 1200.54 [M+Na]⁺. Then the as-obtained Ac-Lys(OAloc)-Gly-Asp(OAll)-Glu(OAll)-Val-Asp(OAll)-PABC (77 mg, 0.065 mmol) and doxorubicin hydrochloride (1.2 eq) were dissolved in anhydrous DMF (8 mL), followed by the addition of DIEA (5.4 eq) and stirring at room temperature in the dark for 16 h. The solvent was then removed under reduced pressure and Ac-Lys(OAloc)-Gly-Asp(OAll)-Glu(OAll)-Val-Asp(OAll)-PABC-DOX (red amorphous solid, 76 mg, 74%) was isolated by preparative HPLC. ESI-MS: m/z 1605.06 [M+Na]⁺. The Ac- Lys(OAloc)-Gly-Asp(OAll)-Glu(OAll)-Val-Asp(OAll)-PABC-DOX (50 mg, 0.032 mmol) and Pd(PPh₃)₄ (0.2 eq) were dissolved in anhydrous DMF (7.6 mL), and acetic acid (20 eq) and tributyltin anhydride (17.3 eq) were then added to the reaction. After stirring at room temperature for 1 h, the solvent was removed under reduced pressure and Ac-Lys-Gly-Asp-Glu-Val-Asp-PABC-DOX (red amorphous solid, 6 mg, 13.6%) deprotected by preservative HPLC was isolated. ESI-MS: m/z 1378.4 [M+H]⁺. The as-obtained Ac-Lys-Gly-Asp- Glu-Val-Asp-PABC-DOX (20 mg, 0.013 mmol) and N-(ε-Ramidocaproxyloxy)succinimide ester (EMCS; 8.26 mg, 26.8 µmol, 2 eq; Pierce, Rockford, IL) were dissolved in anhydrous DMF (1.5 ml; Sigma-Aldrich,St. Louis, MO), followed by the addition of triethylamine (4.64 µl, 2.5 eq; Sigma-Aldrich) and stirring at room temperature for 2 h. The final product was separated by semi-preparative HPLC (Shimadzu, Kyoto, Japan) using an ODS-A 5 µm reversed-phase semi-preparative column (150 mm x 20 mm) on a gradient elution system (water and CH₃CN containing 0.05% TFA as an additive, 20-50% CH₃CN over 50 min, 8 ml/min), and Ac-Lys(EMC)-Gly-Asp-Glu-Val-Asp-PABC-doxorubicin (red amorphous solid 16.7 mg, 73.6%) was obtained whose peaks were monitored at 290 nm. The separation of the final product was confirmed by analytical HPLC (Agilent 1300 series, Agilent Technologies, Santa Clara, CA) using an ODS-A 5 µm analytical column (150 mm x 3 mm; YMC) on a gradient elution system (water and CH₃CN containing 0.05% TFA as an additive, 20-50% CH3CN over 50 min, 8 ml/min). The peaks were monitored by UV detector (214 nm) and fluorescence detector (excitation 470 nm, emission 580 nm) and the purity of the final product was confirmed to be greater than 95%. ESI-MS (m/z): 1593.3 [M + Na]⁺.

### Example 1: Preparation of Ac-Lys-Gly-Asp-Glu-Val-Asp-PABC-DOX (1)

Ac-Lys(OAloc)-Gly-Asp(OAll)-Glu(OAll)-Val-Asp(OAll)-PABC-DOX (10.0 g, 6.4 mmol) and Pd(PPh₃)₄ (1.3 eq) were dissolved in anhydrous DMF (200.0 mL), and hydrochloric acid (27.3 eq) and 4-methylmorpholine (36.0 eq) were added to the reaction. After stirring at room temperature for 2 h, the bis(triphenylphosphine)palladium dichloride was removed by filtration and the reaction solution was injected into a mixture of MeOH (420.0 mL) and dichloromethane (5,520.0 mL) to precipitate the solid to give deprotected Ac-Lys-Gly-Asp-Glu-Val-Asp-PABC-DOX (red amorphous solid, 6.4 g, 73.5%, Pd 60 ppm). The as-obtained Ac-Lys-Gly-Asp-Glu-Val-Asp-PABC-DOX (6.4 g, 4.0 mmol, Pd 60 ppm) was dissolved in PPh₃ (0.1 eq), and hydrochloric acid (0.1 eq) was added to the mixture of MeOH (420.0 mL) and dichloromethane (5,520.0 mL) and stirred for 8 h. The residual Pd was synthesized as PdCl₂(PPh₃)₂, dissolved in MC and MeOH, and filtered to give purified Ac-Lys-Gly-Asp-Glu-Val-Asp-PABC-DOX (6.1 g, 3.9 mmol, Pd 11 ppm).

### Example 2: Preparation of Ac-Lys-Gly-Asp-Glu-Val-Asp-PABC-DOX (2)

Ac-Lys(OAloc)-Gly-Asp(OAll)-Glu(OAll)-Val-Asp(OAll)-PABC-DOX (10.0 g, 6.4 mmol) and Pd(PPh₃)₄ (1.3 eq) were dissolved in anhydrous DMF (200.0 mL), and acetic acid (27.3 eq) and 4-methylmorpholine (36.0 eq) were added to the reaction. After stirring at room temperature for 2 h, the reaction was filtered and the reaction solution was injected into a mixture of chloroform (5,520.0 mL) to precipitate the solid to give deprotected Ac-Lys-Gly-Asp-Glu-Val-Asp-PABC-DOX (red amorphous solid, 6.1 g, 70.0%, Pd 92 ppm).

### Example 3: Preparation of Ac-Lys-Gly-Asp-Glu-Val-Asp-PABC-DOX (3)

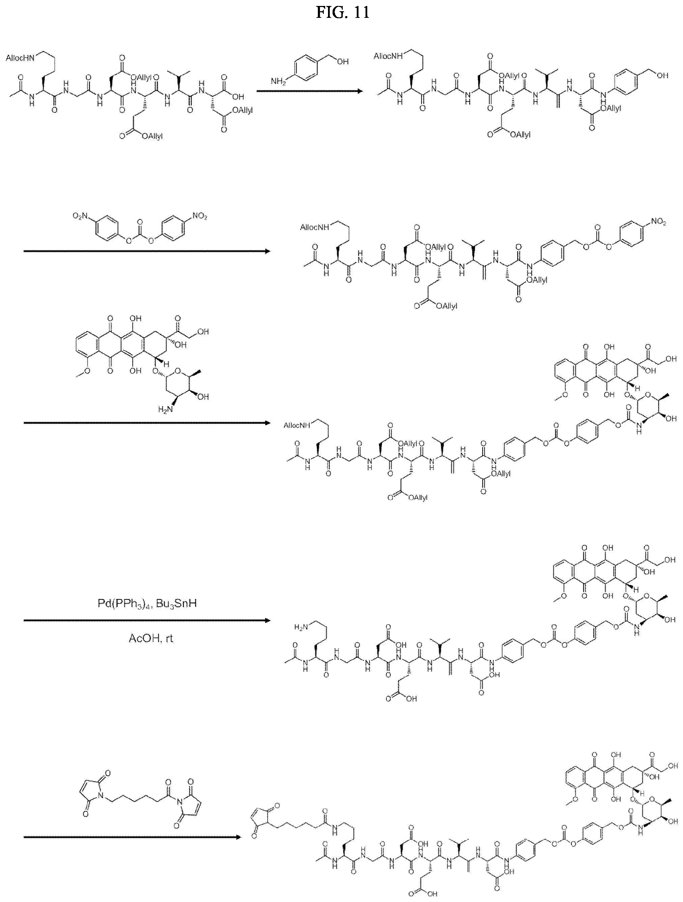

Ac-Lys(OAloc)-Gly-Asp(OAll)-Glu(OAll)-Val-Asp(OAll)-PABC-DOX (10.0 g, 6.4 mmol) and Pd(PPh₃)₄ (1.3 eq) were dissolved in anhydrous DMF (200.0 mL), and acetic acid (27.3 eq) and 4-methylmorpholine (36.0 eq) were added to the reaction. After stirring at room temperature for 2 h, the bis(triphenylphosphine)palladium diacetate was removed by filtration and the reaction solution was injected into a mixture of dichloromethane (5520.0 mL) to precipitate the solid to give deprotected Ac-Lys-Gly-Asp-Glu-Val-Asp-PABC-DOX (red amorphous solid, 6.5 g, 74.5%, Pd 975 ppm). The allylic deprotection reactions of peptides in Examples 2 and 3 above and the prodrug preparation process using acetic acid (AcOH) are shown in FIG. 11, and the variation of residual palladium according to the precipitating solvents used in Examples 2 and 3 is summarized in Table 1 below.

**Table 1**

| Change of residual palladium according to precipitating solvents | | | |
|---|---|---|---|
| | Reagents | Precipitating solvents | Residual Palladium (ppm) |
| Example 2 | AcOH (Acetic acid) | Chloroform | 92 |
| Example 3 | | Dichloromethane | 975 |

### Example 4: Preparation of Ac-Lys-Gly-Asp-Glu-Val-Asp-PABC-DOX (4)

Ac-Lys(OAloc)-Gly-Asp(OAll)-Glu(OAll)-Val-Asp(OAll)-PABC-DOX (10.0 g, 6.4 mmol) and Pd(PPh₃)₄ (1.3 eq) were dissolved in anhydrous DMF (200.0 mL), and hydrochloric acid (27.3 eq) and 4-methylmorpholine (36.0 eq) were added to the reaction. After stirring at room temperature for 2 h, the bis(triphenylphosphine)palladium dichloride was removed by filtration and the reaction solution was injected into a mixture of chloroform (5520.0 mL) to precipitate the solid to give deprotected Ac-Lys-Gly-Asp-Glu-Val-Asp-PABC-DOX (red amorphous solid, 6.2 g, 71.2%, Pd 13 ppm).

### Example 5: Preparation of Ac-Lys-Gly-Asp-Glu-Val-Asp-PABC-DOX (5)

Ac-Lys(OAloc)-Gly-Asp(OAll)-Glu(OAll)-Val-Asp(OAll)-PABC-DOX (50 mg, 0.032 mmol) and Pd(PPh₃)₄ (1.3 eq) were dissolved in anhydrous DMF (1.0 mL), and TFA (15.5 eq) and 4-methylmorpholine (36.0 eq) were added to the reaction. After stirring at room temperature for 2 h, the reaction was filtered. MeOH (2.1 mL) was added to the filtered reaction, and the reaction was injected to a mixture of dichloromethane (27.6 mL) to precipitate the solid to give deprotected Ac-Lys-Gly-Asp-Glu-Val-Asp-PABC-DOX (red amorphous solid, 21 mg, 48.2%, Pd 960 ppm).

### Example 6: Preparation of Ac-Lys-Gly-Asp-Glu-Val-Asp-PABC-DOX (6)

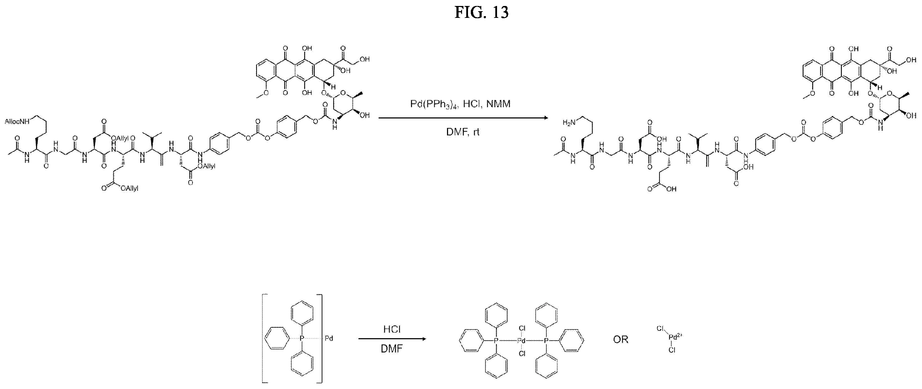

Ac-Lys(OAloc)-Gly-Asp(OAll)-Glu(OAll)-Val-Asp(OAll)-PABC-DOX (50 mg, 0.032 mmol) and Pd(PPh₃)₄ (1.3 eq) were dissolved in anhydrous DMF (1.0 mL), and H₂SO₄ (11.0 eq) and 4-methylmorpholine (36.0 eq) were added to the reaction. After stirring at room temperature for 2 h, the bis(triphenylphosphine)palladium disulfate was removed by filtration and the reaction solution was injected into a mixture of MeOH (2.1 mL) and dichloromethane (27.6 mL) to precipitate the solid to give deprotected Ac-Lys-Gly-Asp-Glu-Val-Asp-PABC-DOX (red amorphous solid, 28 mg, 64.3%, Pd 1,270 ppm). The preparation process of the prodrugs using hydrochloric acid (HCl) between the allyl group deprotection reactions of peptides in Examples 1 and 4 is shown in FIG. 12, and the reaction scheme of tetrakis triphenylphosphine palladium turning into bis triphenylphosphine palladium dichloride in dimethylformamide solvent containing hydrochloric acid is shown in FIG. 13. The variation of residual palladium according to the reagents used in the Reference example, Examples 1, 4 to 5 is summarized in Table 2 below.

**Table 2**

| Change of residual palladium dependent on reagents | | | |
|---|---|---|---|
| | Reagents | Precipitating solvents | Residual Palladium (ppm) |
| Reference example | AcOH (Acetic acid) | - | 3,700 |
| Example 1 | HCl (hydrochloric acid) | Dichloromethane | 60 |
| Example 4 | HCl (hydrochloric acid) | Chloroform | 13 |
| Example 5 | TFA (Trifluoroacetic acid) | Dichloromethane | 960 |
| Example 6 | H₂SO₄ (Sulfuric acid) | Dichloromethane | 1,270 |

### Comparative Example 1: Purification of Ac-Lys-Gly-Asp-Glu-Val-Asp-PABC-DOX (1)

Ac-Lys-Gly-Asp-Glu-Val-Asp-PABC-DOX (20 mg, 0.013 mmol, Pd 3,700 ppm) was dissolved in anhydrous DMF (1.0 mL) and ascorbic acid (1.0 eq) was added to the reaction. After stirring at room temperature for 2 h, the reaction solution was filtered and injected into a mixture of MeOH (2.1 mL) and dichloromethane (27.6 mL) to precipitate the solid to give Ac-Lys-Gly-Asp-Glu-Val-Asp-PABC-DOX (red amorphous solid, 17.5 mg, 87.5%).

### Comparative Example 2: Purification of Ac-Lys-Gly-Asp-Glu-Val-Asp-PABC-DOX (2)

Ac-Lys-Gly-Asp-Glu-Val-Asp-PABC-DOX (20 mg, 0.013 mmol, Pd 3,700 ppm) was dissolved in anhydrous DMF (1.0 mL), and EDTA.2Na (1.0 eq) was added to the reaction. After stirring at room temperature for 2 h, the reaction solution was filtered and injected into a mixture of MeOH (2.1 mL) and dichloromethane (27.6 mL) to precipitate the solid to give Ac-Lys-Gly-Asp-Glu-Val-Asp-PABC-DOX (red amorphous solid, 18.2 mg, 91.0%).

### Comparative Example 3: Purification of Ac-Lys-Gly-Asp-Glu-Val-Asp-PABC-DOX (3)

Ac-Lys-Gly-Asp-Glu-Val-Asp-PABC-DOX (20 mg, 0.013 mmol, Pd 3,700 ppm) was dissolved in anhydrous DMF (1.0 mL), and EDTA.4Na (1.0 eq) was added to the reaction. After stirring at room temperature for 2 h, the reaction solution was filtered and injected into a mixture of MeOH (2.1 mL) and dichloromethane (27.6 mL) to precipitate the solid to give Ac-Lys-Gly-Asp-Glu-Val-Asp-PABC-DOX (red amorphous solid, 18.0 mg, 90.0%).

### Comparative Example 4: Purification of Ac-Lys-Gly-Asp-Glu-Val-Asp-PABC-DOX (4)

Ac-Lys-Gly-Asp-Glu-Val-Asp-PABC-DOX (20 mg, 0.013 mmol, Pd 3,700 ppm) was dissolved in anhydrous DMF (1.0 mL) and DTPA (1.0 eq) was added to the reaction. After stirring at room temperature for 2 h, the reaction solution was filtered and injected into a mixture of MeOH (2.1 mL) and dichloromethane (27.6 mL) to precipitate the solid to give Ac-Lys-Gly-Asp-Glu-Val-Asp-PABC-DOX (red amorphous solid, 16.9 mg, 84.5%). The variation of residual palladium according to the chelating agent used in Examples 7 to 10 is summarized in Table 3 below.

**Table 3**

| Change of residual palladium dependent on chelating agents | | |
|---|---|---|
| | Chelating agents | Residual Palladium (ppm) |
| Comparative example 1 | Ascorbic acid | 3,570 |
| Comparative example 2 | EDTA.2Na (dehydrated ethylenediaminetetraacetic acid disodium) | 3,558 |
| Comparative example 3 | EDTA.4Na (dehydrated ethylenediaminetetraacetic acid disodium) | 3,615 |
| Comparative example 4 | DTPA (diethylenetriamine pentaacetic acid) | 3,458 |

### Comparative Example 5: Purification of Ac-Lys-Gly-Asp-Glu-Val-Asp-PABC-DOX (5)

Ac-Lys-Gly-Asp-Glu-Val-Asp-PABC-DOX (20 mg, 0.013 mmol, Pd 3,700 ppm) was dissolved in anhydrous DMF (1.0 mL), and NUCHAR SA-20 (2.0 mg) was added to the reaction. After stirring at room temperature for 2 h, the reaction solution was filtered and injected into a mixture of MeOH (2.1 mL) and dichloromethane (27.6 mL) to precipitate the solid to give Ac-Lys-Gly-Asp-Glu-Val-Asp-PABC-DOX (red amorphous solid, 18.0 mg, 90.0%).

### Comparative Example 6: Purification of Ac-Lys-Gly-Asp-Glu-Val-Asp-PABC-DOX (6)

Ac-Lys-Gly-Asp-Glu-Val-Asp-PABC-DOX (20 mg, 0.013 mmol, Pd 3,700 ppm) was dissolved in anhydrous DMF (1.0 mL), and NUCHAR HD (2.0 mg) was added to the reaction. After stirring at room temperature for 2 h, the reaction solution was filtered and injected into a mixture of MeOH (2.1 mL) and dichloromethane (27.6 mL) to precipitate the solid to give Ac-Lys-Gly-Asp-Glu-Val-Asp-PABC-DOX (red amorphous solid, 17.7 mg, 88.5%).

### Comparative Example 7: Purification of Ac-Lys-Gly-Asp-Glu-Val-Asp-PABC-DOX (7)

Ac-Lys-Gly-Asp-Glu-Val-Asp-PABC-DOX (20 mg, 0.013 mmol, Pd 3,700 ppm) was dissolved in anhydrous DMF (1.0 mL), and NUCHAR GC (2.0 mg) was added to the reaction. After stirring at room temperature for 2 h, the reaction solution was filtered and injected into a mixture of MeOH (2.1 mL) and dichloromethane (27.6 mL) to precipitate the solid to give Ac-Lys-Gly-Asp-Glu-Val-Asp-PABC-DOX (red amorphous solid, 17.8 mg, 89.0%). The variation of residual palladium according to the activated carbons used in Examples 11 to 13 is summarized in Table 4 below.

**Table 4**

| Change of residual palladium dependent on activated carbons | | |
|---|---|---|
| | Activated carbons | Residual palladium (ppm) |
| Comparative example 5 | NUCHAR SA-20 (meadwestvaco corporation) | 3,570 |
| Comparative example 6 | NUCHAR HD (meadwestvaco china holding co ltd) | 3,458 |
| Comparative example 7 | NUCHAR GC (meadwestvaco china holding co ltd) | 3,431 |

### Comparative Example 8: Purification of Ac-Lys-Gly-Asp-Glu-Val-Asp-PABC-DOX (8)

Ac-Lys-Gly-Asp-Glu-Val-Asp-PABC-DOX (20 mg, 0.013 mmol, Pd 3,700 ppm) was dissolved in anhydrous DMF (1.0 mL) and Celite 545 (2.0 mg) was added to the reaction. After stirring at room temperature for 2 h, the reaction solution was filtered and injected into a mixture of MeOH (2.1 mL) and dichloromethane (27.6 mL) to precipitate the solid to give Ac-Lys-Gly-Asp-Glu-Val-Asp-PABC-DOX (red amorphous solid, 15.5 mg, 77.5%).

### Comparative Example 9: Purification of Ac-Lys-Gly-Asp-Glu-Val-Asp-PABC-DOX (9)

Ac-Lys-Gly-Asp-Glu-Val-Asp-PABC-DOX (20 mg, 0.013 mmol, Pd 3,700 ppm) was dissolved in anhydrous DMF (1.0 mL), and silica gel (2.0 mg) was added to the reaction. After stirring at room temperature for 2 h, the reaction solution was filtered and injected into a mixture of MeOH (2.1 mL) and dichloromethane (27.6 mL) to precipitate the solid to give Ac-Lys-Gly-Asp-Glu-Val-Asp-PABC-DOX (red amorphous solid, 14.9 mg, 74.5%). The variation of residual palladium with the use of Celite 545 or Silica gel in Examples 14 and 15 is summarized in Table 5 below.

**Table 5**

| Change of residual palladium dependent on the use of Celite 545 or silica gel | | |
|---|---|---|
| | Used material | Residual palladium (ppm) |
| Comparative example 8 | Celite 545 | 3,680 |
| Comparative example 9 | Silica gel | 3,696 |

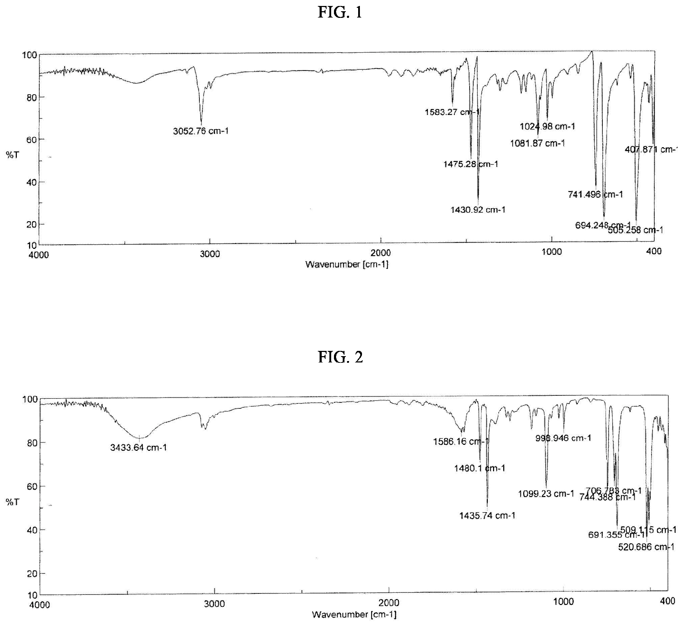

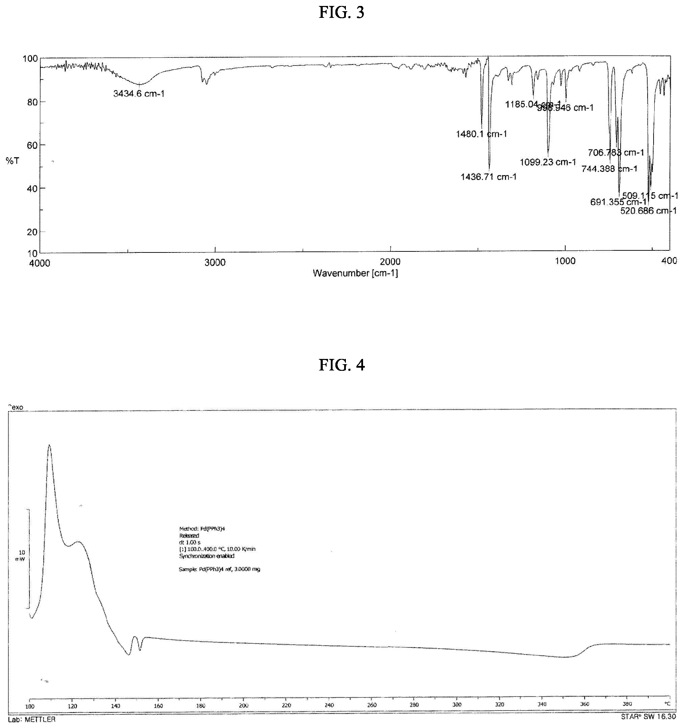

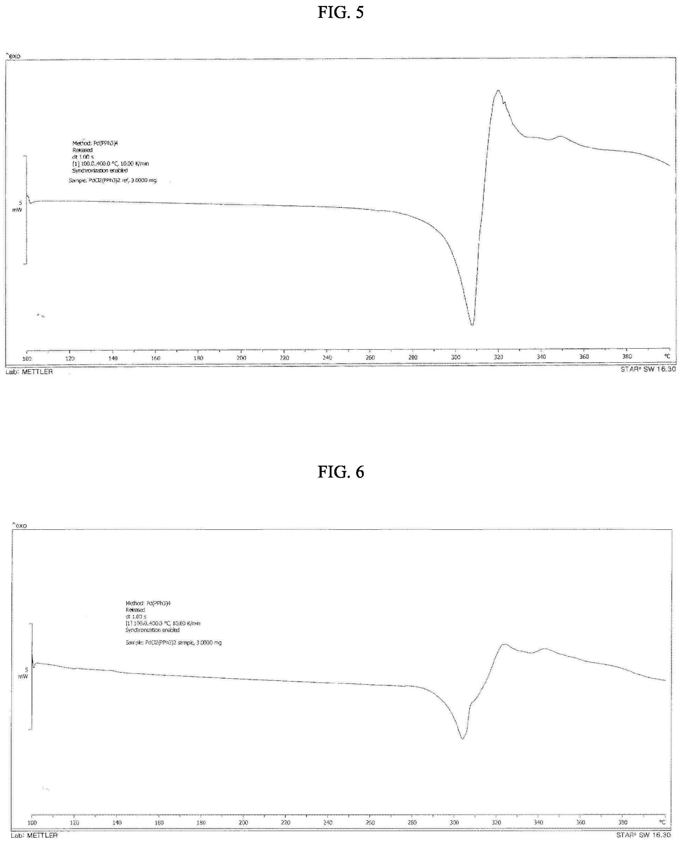

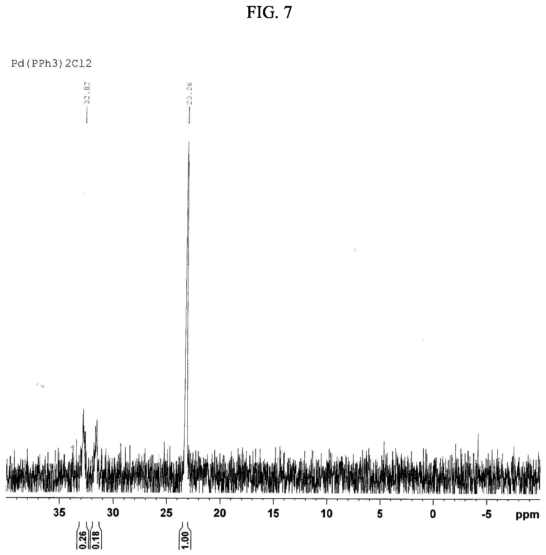

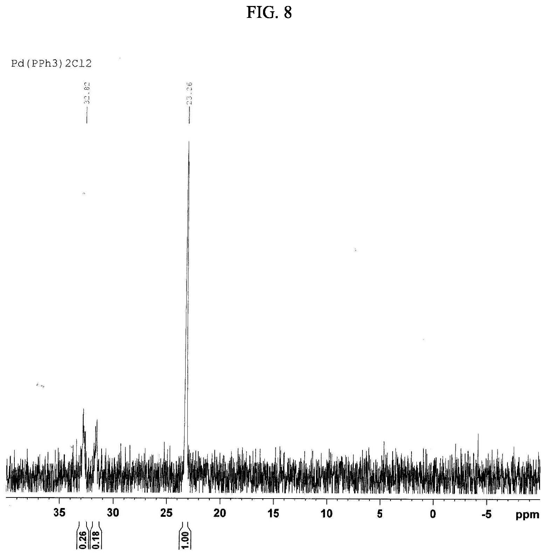

In conclusion, residual palladium can be removed during chloroform crystallization as shown in the analytical results in Tables 1 and 2 above. However, due to its classification as a genotoxic substance, chloroform residues must be controlled to 3 ppm or less. Also, a single purification is required to control residual palladium to 25 ppm or less. Furthermore, as shown in the analytical results in Tables 2 to 5 above, palladium was not removed in all experiments except when hydrochloric acid was used as a reagent. In addition, the palladium filtrate precipitated from the deprotection reaction of the allylic group in the peptide spacer was identified as bis-triphenylphosphine palladium dichloride, as shown in Table 5. Bis triphenylphosphine palladium dichloride exhibited characteristic peaks around 1480, 1436, 1099, 998, 744, 706, 691, 520, and 509 (cm⁻¹) in the infrared absorption spectrum with a margin of error of ± 2 cm⁻¹). In addition, the precipitated palladium filtrate and bis-triphenylphosphine palladium dichloride exhibited the same peaks in ³¹P-NMR (162 MHz, CDC1₃) (FIGS. 1 to 9). [δ23.25(s)]

The present invention has been described with reference to the embodiments described above, but these are exemplary only, and one of ordinary skill in the art will understand that various modifications and other equally valid embodiments are possible from them. The true scope of technical protection of the invention should therefore be determined by the technical ideas of the appended claims of the patent.

## Claims

1. A method of removing and recovering high purity palladium compounds from the preparation process of prodrug conjugates, comprising
preparing an allyl group-protected precursor of a prodrug conjugate in which an anticancer compound linked to a peptide linker that is to be cleaved by a proteolytic enzyme;
preparing a deprotected reaction solution in which the allyl groups of the precursor have been removed by dissolving the precursor and tetrakis triphenylphosphine palladium (Pd(PPh₃)₄) in an anhydrous organic solvent;
removing bis(triphenylphosphine)palladium dichloride (PdCl₂₍(PPh₃)₂) prepared by a reaction between Pd(PHh₃)₄ and hydrochloric acid by adding hydrochloric acid and a catalyst to the deprotected reaction solution, stirring at room temperature, and filtering a reactant; and
precipitating an amorphous solid by adding a mixture including an organic solvent and a precipitating solvent to the deprotected reaction solution in which PdCl₂₍(PPh₃)₂ is removed.

2. The method according to claim 1, further comprising purifying the prodrug conjugate by adding the obtained prodrug conjugate to a reaction solution comprising triphenylphosphine, hydrochloric acid and a precipitating solvent.

3. The method according to claim 1, wherein the prodrug conjugate is maleimide-KGDEVD-PABC-doxorubicin.

4. The method according to claim 1, wherein the catalysts is selected from the group comprising 4-methylmorpholine, morpholine, 4-ethylmorpholine, pyrrolidine, properidine, N-methylpiperidine, N-methylpyrrolidine, piperazine, and imidazoliden.

5. The method according to claim 1, wherein the precipitating solvent is dichloromethane or chloroform.

6. The method according to claim 1, wherein the anhydrous organic solvent is dimethylformamide, dichloromethane, or chloroform.

7. The method according to claim 1, wherein the organic solvent is ethanol, methanol, or acetone.
